# EUROPEAN PATENT APPLICATION

(11) **EP 2 316 465 A1**
(43) Date of publication of application: **04.05.2011**
(21) Application number: 09290786.4
(22) Date of filing: 15.10.2009
(51) Int. Cl.: A61K 35/62, A61P 19/02, A61P 31/04

(54) **Use of extract of leeches' saliva as anti-bacterial agent for the manufacture of different compositons**

(71) Applicant: CENTRE NATIONAL DE LA RECHERCHE SCIENTIFIQUE, 75794 Paris Cedex 16 (FR)
(72) Inventor: Tasiemski, Aurélie, 59110 LA MADELEINE (FR); Salzet, Michel, 59830 Bourghelles (FR)
(74) Representative: Mouget-Goniot, Claire

(57) **Abstract**

The present invention relates to the use of extract of leeches' saliva as anti-bacterial agent for the manufacture of different compositions, such as a pharmaceutical composition, a cosmetic composition or a cleaning product.

## Description

The present invention relates to the use of extract of leeches' saliva as anti-bacterial agent for the manufacture of different compositions.

Today, the infection by resistant bacteria is a more and more serious public sanitary problem. Many bacterial lines became resistant to most kinds of currently disposable antibiotic treatments. It becomes a real challenge to find new antibacterial agents to fight against resistant bacteria, particularly in the frame of fighting against nosocomial infections. The market for new antibacterial agents is estimated to be 5 billion dollars in US.

In industrial domain, elimination of bacteria is effected, up to now, by long, heavy and expensive processes, such as cleaning operation and large scale disinfection. However, said processes are sometimes not efficient. Furthermore, since more and more drastic norms, such as norms "REACH" or "BIOCIDES", enter in force, the number of usable products considerably decrease. For example, FDA has recently limited the use of certain components, such as Triclosan, that generates important bacterial resistance. Furthermore, most of antibacterial agents used up to now are not natural and able to provoke environmental problems, linked to the difficulty of elimination of these agents.

Food infection is another public healthy problem linked to the contamination of the food by pathogenic bacteria such as *Salmonellae, Listeria monocytogenes, Staphylococcus aureus, Bacillus cereus, Enterococcus fecalis* or *Campylobacter jejuni.* The incidence of food infection, that is frequent in the developing countries, is also increasing in the developed countries.

Recently, researchers have turned to new biological source based on new mechanisms, and subsequently several new antibacterial agents that would not provoke bacterial resistance have been found.

The antimicrobial peptides (AMP) is a kind of natural antibiotic, not immunogenic and produced by plants or animals having a special mode of action and activity. They generally react at level of bacterial membranes and do not favor the apparition of resistant bacterial lines. Among the AMP present in the market, *recombinant plectasin,* for example, is used for fighting against the Gram+ bacteria, particularly against *Streptococcus pneumonia,* a kind of bacteria known for its resistance to classic antibiotherapy. Other AMPs, such as the *Pexiganan,* are today in clinical phase 3. Said compound, derived from magainin of amphibians, targets the treatment of impetigo and stomach ulcer. Other compoounds may be cited like *isaganan,* derived from porcine protegrin for the treatment of oesophagitis or stomach ulcer, or *Neuprex* issued from human protein rBP123, or still *Ominagan,* issued from indolicin, for the treatment of infection linked to the use of catheters.

Recently, in the frame of studies relating to the development of diagnostic tools for osteoarthritis, the Inventors of the present invention have surprisingly found that osteoarthritis has a bacterial origin. In the previous works, the Inventors have found 4 new antimicrobial peptides in central nervous system of leeches. Now, the Inventors find that antibacterial agents are also present in leeches' saliva.

The history of use of leeches of species *Hirudo medicinalis* in medicine goes back to the 18^{th} Egyptian dynasty. In the 19^{th} century in France, leeches have been used in the treatment of pharyngitis, ophthalmic problems, obesity, and mental disorders. During the treatment, leeches were directly placed on the abdomen of patient. Later, the treatment by leeches was failing into disuse until the fifties of the last century. For example, in 1948, leeches were used by P.Durand, P.Viard and R. Vendel in the treatment of asthma.

Today, with the development of microsurgery, leeches are generally used in hospitals during a plastic or traumatological surgery to resolve many problems due to the insufficiency of venous drainage. The survival of a reimplanted tissue depends on the efficiency of venous return. However, it is very difficult to establish a venous return since it is more difficult to stitch a vein than an artery. Certain surgeons then consider local application of leeches to resolve this problem. By their suction, leeches stimulate drainage of tissue in danger of necrosis. They favor also the restoration of capillaries match between the faces of a wound where a surgical surture is technically impossible.

In 2003, German researchers found that leech therapy can help relieve symptoms in patients with osteoarthritis of the knee by directly placing leeches on the knees of patients (Michalsen et al., Ann intern Med. 2003; 139: 724-730).

However, leech therapy used up to now, using living organisms, presents a potential infection risk, especially when there is an enter gate, such as a leech bite. In 1983, Whitlock et al. (Br J Plast Surg 1983; 36: 240-3) suggested for the first time the possibility of subcutanous infection caused by *Aeromonas hydrophila,* a kind of Gram negative aerobic bacteria, by the intermediate of leech therapy. This kind of bacteria exists in intestinal flora of leeches and digests the red blood cells taken by leeches. This bacterium is also linked to two types of gastroenteritis. The first type is a disease similar to cholera, which causes rice-water diarrhea. The other type of disease is dysenteric gastroenteritis, which causes loose stools filled with blood and mucus.

*Aeromonas hydrophila* is not the only pathogen presents in leeches that can provoke an infection. A small quantity of Gram negative bacillus including *Pseudomonas* and a tiny proportion of anaerobic germ can also be found on the surface of leeches.

Disinfection of leeches before leech therapy is generally effected by immersion of leeches in antibiotic solution and then rinsing by sterile water. However, this method can not always efficiently disinfect leeches. Sometimes, it causes also allergy in patient and affects the suction capacity of leeches.

The patent application FR2834292 reports the use of leeches extract as anticoagulant, antiinflammatory or thrombolytic agents.

The patent application FR2834293 presents the use of extract prepared from starved leeches as antinarcotic, antistress or antiinflammatory agent.

The patent application FR2843883 concerns non-irritating leech extract, having antiinflammatory and/or antipsoriatic activity, obtained by suspending homogenized crude extract in saline, incubating and recovering supernatant.

But these prior patent applications have not provided any information about antibacterial activity of leeches extract, in particular saliva extract.

Consequently, there is an important need to find a new, safe, natural antibacterial agent that on the one hand does not provoke bacterial resistance and on the other hand does not have the drawback of living leeches.

The first aspect of the present invention relates to the use of an extract of leeches for the manufacture of an anti-bacterial composition, selected from the group consisting of a pharmaceutical composition, a cosmetic composition or a cleaning product.

According to the present invention, extract of leeches means homogenised tissues from the whole leech as well as any part of the leech, in particular secreted salivary glands, said homogenised tissues showing antibacterial activity.

Leeches used in the present invention can be the species chosen from the Hirudinidea *e.g. Hirudo medicinalis, Hirudo verbena, Hirudinaria mallinensis, Haemopis marmorata, Macrobdella decora.*

In an advantageous embodiment, the pharmaceutical composition is useful for the treatment of diseases selected from osteoarthritis, foodborne illnesses caused by a pathogenic bacteria or nosocomial infections.

According to the present invention, foodborne illnesses mean food infection caused by the presence of bacteria, for example, *Campylobacter jejuni, Salmonellas, Listeria monocytogenes, Staphylococcus aureus, Bacillus cereus, Enterococcus fecalis,* or other microbes which infect the body after consumption of food.

According to the present invention, nosocomial infections mean infections which are the result of treatment in a hospital or a healthcare service unit, but secondary to the patient's original condition. Infections are considered nosocomial if they first appear 48 hours or more after hospital admission or within 30 days after discharge. This type of infection is also known as a hospital-acquired infection (or more generically healthcare-associated infection).

Nosocomial infections are often caused by Gram-positive bacteria, such as *Staphylococcus aureus,* Methicillin Resistant *Staphylococcus aureus, Clostridium difficile,* Vancomycin-resistant *Enterococcus,* or Gram-negative bacteria, such as *Pseudomonas aeruginosa, Acinetobacter baumannii, Legionella,* or mycobacteria As nosocomial infections, we can recite as example without limitation, ventilator associated pneumonia, hospital-acquired tuberculosis, hospital-acquired urinary tract infection, hospital-acquired pneumonia or hospital-acquired gastroenteritis.

In another advantageous embodiment, the antibacterial agents of the present invention are active against the bacteria of class II or class III.

The biological agents are classified in 4 classes according to the importance of risk of propagation that they represent.

Class I is the biological agent which is not susceptible to provoke a disease in human. Class II is the biological agent which can provoke a disease in human and constitute a danger for the public, but which propagation in the community is improbable. There is generally a prophylaxis or an efficient treatment for this disease.

Class III is the biological agent which can provoke a disease in human and constitute a serious danger for the public, and which has a risk of propagation in community. However there is generally a prophylaxis or an efficient treatment for this disease.

Class IV is the biological agent which provokes diseases in human and constitutes a serious danger for the pubic, and which has a high risk of propagation in community. There is not generally any prophylaxis or efficient treatment for these diseases.

In a more advantageous embodiment, bacteria of class II or class III are selected from the group consisting of *Acinetobacter thyphimirium, Salmonella thyphimirium, Alcaligenes faealis, Haemophillus influenzae, Shewanella, Escherichia coli, Aerococcus viridans, Microcuccus luteus, Staphylocuccus, saprophyticus.*

Another embodiment of the invention is a cleaning product selected from the group consisting of a general household disinfectant, a window cleaner, a bathroom cleaner, a kitchen cleaner, a floor cleaner, a laundry detergent, a cleaning supply, a fruit and a vegetable wash, and a fabric softener.

In an advantageous embodiment, the extract of leeches used in the present invention is an extract of saliva of leeches. According to the invention an extract of saliva means crude saliva, purified saliva or any isolation of the saliva so long as it shows the antibacterial activity.

Saliva of leeches may be extracted according to any conventional method, for example, the method of Bligh. E.J. and Dyer. H.J. (Biochem, physiol. 37, 911-917 (1959)).

The second aspect of the present invention relates to purified leeches saliva extract for the manufacture of an anti-bacterial composition, selected from the group consisting of a pharmaceutical composition, a cosmetic composition or a cleaning product.

Purified leeches saliva extract may be obtained according to any conventional process, for example by the following method:
- extract leeches saliva in large quantity according to a conventional method,
- sterilize leeches saliva by any conventional method, such as by UV, and optionally
- purify leeches saliva by any conventional method, such as by chromatography.

In an advantageous embodiment of the invention, the pharmaceutical composition containing purified leeches saliva extract is useful for the prevention or the treatment of diseases selected from osteoarthritis, foodborne illnesses caused by a pathogenic bacteria or nosocomial infections.

In a more advantageous embodiment, the nosocomial infections are caused by a kind of bacteria selected from the group consisting of bacteria (gram +, gram -) or mycobacteria.

In another more advantageous embodiment, the anti-bacterial composition acts against the bacteria of Class II and/or of Class III.

In a particularly more advantageous embodiment, the bacteria is selected from the group consisting of *Acinetobacter thyphimirium, Salmonella thyphimirium, Alcaligenes faealis, Haemophillus influenzae, Shewanella, Escherichia coli, Aerococcus viridans, Microcuccus luteus, Staphylocuccus, saprophyticus, Helicobacter pyroli.*

In another embodiment, the cleaning product containing purified leeches saliva extract is selected from the group consisting of a general household disinfectant, a window cleaner, a bathroom cleaner, a kitchen cleaner, a floor cleaner, a laundry detergent, a cleaning supply, a fruit and a vegetable wash, and a fabric softener.

In another embodiment, purified leeches saliva extract is useful for the manufacture of a drug.

Another aspect of the present invention relates also to the composition comprising leeches saliva extract as anti-bacterial agent, wherein said composition is selected from the group consisting of a pharmaceutical composition or a cleaning product.

In a particular embodiment of this aspect, pharmaceutical composition comprising leeches saliva extract as anti-bacterial agent is for the prevention or the treatment of diseases selected from osteoarthritis, foodborne illness caused by a pathogenic bacteria or nosocomial infections.

According to the invention, pharmaceutical composition may be administrated by topical route, enteral route or parenteral route by injection or infusion.

Said pharmaceutical composition can be manufactured in a liquid form, such as an injection, a spray, or in a solid form, such as tablets, capsules.

Said pharmaceutical composition can also contain a pharmaceutical acceptable carrier, or others active substances, in particular other antibacterial agents, or diluents, emulsifiers, adjuvants and vehicles as desired.

In another particular embodiment of this aspect, cleaning product comprising leeches saliva extract as anti-bacterial agent is selected from the group consisting of a general household disinfectant, a window cleaner, a bathroom cleaner, a kitchen cleaner, a floor cleaner, a laundry detergent, a cleaning supply, a fruit and a vegetable wash, and a fabric softener.

Said cleaning product can comprise also any conventional antistatic agents and surfactants.

Another object of the invention is a method for treating or preventing diseases selected form osteoarthritis, foodborne illnesses caused by a pathogenic bacteria or nosocomial infections, said method comprising administration of an extract of leeches to a patient in need thereof.

The invention is illustrated by the following example.

### Example

### Test of antimicrobial activity of crude extract of leeches' saliva

50µl of crude extract of leeches' saliva are added to 500µl of culture of different bacteria in exponential phase. The tested bacteria are listed in the following table. The bactericidal activity of crude extract of leeches' saliva in different bacterial solution is tested during 24 hours. When activity was detected, all the bacteria are killed in less than 24 hours.

| **Gram-negative bacteria** | |
|---|---|
| *Acinetobacter baumannii* | ++ |
| *Salmonella thyphimirium* | + |
| *Alcaligenes faecalis* | + |
| *Haemophillus influenzae* | + |
| *Shewanella* | ++ |
| *Echerichia coli* | + |
| | |

| **Gram-positive bacteria** | |
|---|---|
| *Aerococcus viridans* | ++ |
| *Micrococcus luteus* | ++ |
| *Staphylococcus saprophyticus* | + |
| *Staphylococcus aureus* | + |
| *(*++*) high activity detected; (*+*) low activity detected* | |

## Claims

1. Use of extract of leeches for the manufacture of an anti-bacterial composition, selected from the group consisting of a pharmaceutical composition, a cosmetic composition or a cleaning product.

2. Use according to claim 1, wherein the pharmaceutical composition is for the treatment of diseases selected from osteoarthritis, foodborne illnesses caused by a pathogenic bacteria or nosocomial infections.

3. Use according to claim 2, wherein the nosocomial infection is caused by bacteria selected from the group consisting of bacteria (gram +, gram -) or mycobacteria.

4. Use according to any one of claims 1 to 3, wherein said anti-bacterial acts against the bacteria of Class II and/or of Class III.

5. Use according to claim 4, wherein said bacterium is selected from the group consisting of *Acinetobacter thyphimirium, Salmonella thyphimirium, Alcaligenes faealis, Haemophillus influenzae, Shewanella, Escherichia coli, Aerococcus viridans, Microcuccus luteus, Staphylocuccus, saprophyticus, Helicobacter pylori.*

6. Use according to claim 1, wherein the said cleaning product is selected from the group consisting of a general household disinfectant, a window cleaner, a bathroom cleaner, a kitchen cleaner, a floor cleaner, a laundry detergent, a cleaning supply, a fruit and a vegetable wash, and a fabric softener.

7. Use according to claims 1 to 6, wherein said extract of leeches is an extract of saliva of leeches.

8. Purified leeches saliva extract for the manufacture of an anti-bacterial composition, selected from the group consisting of a pharmaceutical composition, a cosmetic composition or a cleaning product.

9. Purified leeches saliva extract according to claim 8, wherein said pharmaceutical composition is for the prevention or the treatment of diseases selected from osteoarthritis, foodborne illnesses caused by a pathogenic bacteria or nosocomial infections.

10. Purified leeches saliva according to claim 9, wherein the nosocomial infection is caused by bacteria selected from the group consisting of bacteria (gram +, gram -) or mycobacteria.

11. Purified leeches saliva according to claims 9 and 10, wherein said anti-bacterial composition acts against the bacteria of Class II and/or of Class III.

12. Purified leeches saliva according to claim 11, wherein said bacteria is selected from the group consisting of *Acinetobacter thyphimirium, Salmonella thyphimirium, Alcaligenes faealis, Haemophillus influenzae, Shewanella, Escherichia coli, Aerococcus viridans, Microcuccus luteus, Staphylocuccus, saprophyticus, Helicobacter pylori.*

13. Purified leeches saliva according to claim 8, wherein the said cleaning product is selected from the group consisting of a general household disinfectant, a window cleaner, a bathroom cleaner, a kitchen cleaner, a floor cleaner, a laundry detergent, a cleaning supply, a fruit and a vegetable wash, and a fabric softener.

14. Purified leeches saliva extract for the manufacture of a drug.

15. Composition comprising leeches saliva extract as anti-bacterial agent, wherein said composition is selected from the group consisting of a pharmaceutical composition or a cleaning product.

16. Pharmaceutical composition according to claim 15 for its use in the prevention or the treatment of diseases selected from osteoarthritis, foodborne illnesses caused by a pathogenic bacteria or nosocomial infections.

17. Pharmaceutical composition according to claim 16, wherein said pharmaceutical composition is administrated by topical route, enteral route or parenteral foute by injection or infusion.

18. Composition according to claim 15, wherein said cleaning product is selected from the group consisting of a general household disinfectant, a window cleaner, a bathroom cleaner, a kitchen cleaner, a floor cleaner, a laundry detergent, a cleaning supply, a fruit and a vegetable wash, and a fabric softener.
